(19) Europäisches Patentamt
European Patent Office
Office européen des brevets

(11) Veröffentlichungsnummer: **0 041 043**
**B1**

(12) # EUROPÄISCHE PATENTSCHRIFT

(45) Veröffentlichungstag der Patentschrift: **09.11.83**

(21) Anmeldenummer: **81810177.6**

(22) Anmeldetag: **07.05.81**

(51) Int. Cl.³: **C 07 C 1/26**, C 07 C 15/14, C 07 C 15/24, C 07 C 15/52, C 07 C 25/24, C 07 C 43/215, C 07 C 47/548, C 07 C 79/10, C 07 C 121/64, C 07 D 239/26, C 07 C 17/26

(54) **Verfahren zur Herstellung von Alkenylbenzol- oder -naphthalin-derivaten.**

(30) Priorität: **13.05.80 CH 3731/80**

(43) Veröffentlichungstag der Anmeldung: **02.12.81 Patentblatt 81/48**

(45) Bekanntmachung des Hinweises auf die Patenterteilung: **09.11.83 Patentblatt 83/45**

(84) Benannte Vertragsstaaten: **CH DE FR GB IT LI**

(56) Entgegenhaltungen:
**US - A - 3 922 299**

**Chemical Abstracts Band 73, Nr. 11, 1970 Columbus, Ohio, USA J. TSUJI et al "Nonenes, octenes, styrene, and heptenes" Seite 286, Spalte 1, Abstract Nr. 55611n**
**Derwent Japanese Patents Report, Band 7, Nr. 5, 1968 TOYO RAYON CO "Manufacture of olefins" Teil 4, Seite 1**
**Chemical Abstracts bBand 92, Nr. 13, 1980 Columbus, Ohio, USA A. BIAVATI et al. "Palladium or nickel-catalyzed benzoylation and phenylation of methyl acrylate" Seite 593, Spalte 2, Abstract Nr. 110202z**

(73) Patentinhaber: **CIBA-GEIGY AG, Patentabteilung Postfach, CH-4002 Basel (CH)**

(72) Erfinder: **Blaser, Hans-Ulrich, Dr., Brücklismattstrasse 18, CH-4107 Ettingen (CH)**
Erfinder: **Reinehr, Dieter, Dr., Wolfsheule 10, D-7842 Kandern (DE)**
Erfinder: **Spencer, Alwyn, Dr., Schützengraben 15, CH-4051 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von Alkenylbenzol- oder -naphthalinderivaten

Die vorliegende Erfindung betrifft ein neues Verfahren zur Herstellung von Alkenylbenzol- oder -naphthalinderivaten.

Aus der US-Patentschrift 3 922 299 ist bekannt, daß man vinylisch oder allylisch substituierte organische Verbindungen, besonders Zimtsäure und Zimtsäureester, durch katalytische Umsetzung von entsprechenden Halogeniden mit aktivierten Olefinen, wie Acrylsäuremethylester, in Gegenwart von tertiären Aminen herstellen kann. Als Katalysatoren werden bevorzugt Gemische von Palladiumacetat und Triphenylphosphin oder Tri-(orthotolyl)-phosphin verwendet. Die Umsetzung kann auch so vorgenommen werden, daß man zuerst aus dem Halogenid und dem Katalysatorsystem einen Komplex bildet und diesen anschließend in Gegenwart eines tertiären Amins mit dem Olefin reagieren läßt. Andererseits ist bekannt, daß die Umsetzung von Benzoylchlorid mit Acrylsäuremethylester in Gegenwart stöchiometrischer Mengen eines Nickel(0)-Katalysators bei Nachbehandlung des Reaktionsgemisches mit Jod in Methanol zur Bildung von trans-3-Benzoylacrylsäuremethylester führt. Als Nebenprodukt fällt dabei Zimtsäuremethylester an. Durch Umsetzung eines Komplexes aus Benzoylpalladiumchlorid und Triphenylphosphin mit Acrylsäuremethylester bei 70 bis 85°C in Gegenwart von Triäthylamin erhält man Zimtsäuremethylester als Hauptprodukt und Benzoylacrylsäuremethylester als Nebenprodukt. Werden das Palladium und das Triphenylphosphin nur in katalytischen Mengen eingesetzt, so verschiebt sich das Reaktionsgleichgewicht zu Gunsten der Bildung des Benzoylacrylsäuremethylesters (Gewichtsverhältnis von Benzoylacrylsäuremethylester zu Zimtsäuremethylester = ca. 8,3 : 1) [vgl. Transition Met. Chem. 2,270 (1977) und 4,398 (1979)]. Schließlich ist aus Synthesis, 777 (1977) bekannt, daß die Umsetzung von aromatischen Säurehalogeniden mit 1-Alkinen unter Pd-Katalyse ohne Decarbonylierung zu Alkinylketonen führt.

Es wurde nun gefunden, daß man Verbindungen der Formel I

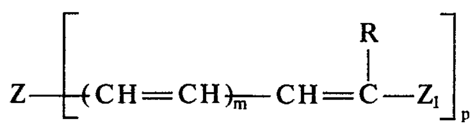

(I)

worin

- Z bei p = 1 gegebenenfalls substituiertes Phenyl oder Naphthyl und bei p = 2 gegebenenfalls substituiertes Phenylen, Naphthylen oder p-Biphenylen,
- $Z_1$ gegebenenfalls substituiertes Phenyl oder Naphthyl,
- R Wasserstoff oder $C_{1-4}$-Alkyl,
- m Null oder die Zahl 1 und
- p die Zahl 1 oder 2 bedeuten,

dadurch herstellen kann, daß man eine Verbindung der Formel II

$$Z-[(CH=CH)_m-CO-X]_p \qquad (II)$$

worin Z, m und p die unter Formel I angegebene Bedeutung haben und X Chlor, Brom oder Jod darstellt, in Gegenwart einer Base und unter Zusatz von Palladium-Metall oder von Palladiumverbindungen, die unter den Reaktionsbedingungen phosphorfreie labile Palladium(0)-Verbindungen bilden, als Katalysator mit einer Verbindung der Formel III oder, bei p = 2, auch einem Gemisch von zwei verschiedenen Verbindungen der Formel III

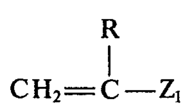

(III)

worin für R und $Z_1$ das unter Formel I angegebene gilt, umsetzt.

Nach dem erfindungsgemäßen Verfahren lassen sich die Verbindungen der Formel I auf einfache, wirtschaftliche Weise und unter Verwendung von leicht zugänglichen Ausgangsprodukten herstellen. Dabei ist es überraschend, daß die Reaktion selektiv unter Decarbonylierung der Säurehalogenide der Formel II verläuft.

Die in Gruppen Z und $Z_1$ vorkommenden Substituenten sind solche, die unter den Reaktionsbedingungen inert sind. Die genannten Gruppen Z und $Z_1$ können ein- oder mehrfach substituiert sein. Dabei können die Substituenten gleich oder verschieden sein.

Als Substituenten an Gruppen Z oder $Z_1$ kommen z. B. in Betracht: Halogenatome, Formyl, $-CH(OCH_3)_2$, $-CH(OC_2H_5)_2$,

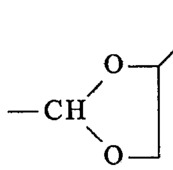 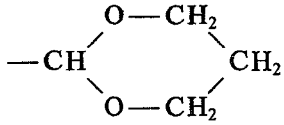 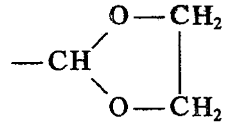

$-CH{=}CH-CN$, $-CH{=}CH-COO-C_{1-4}$-Alkyl, $C_{1-10}$-Alkyl-, $C_{1-16}$-Alkoxy-, Phenoxy-, Di($C_{1-10}$-alkyl)amino-, Nitro-, Cyano-, $-CH_2Cl-$, Trifluormethyl-, Benzyl, $C_{1-4}$-Alkylsulfonyl-, $-CO-C_{1-10}$-Alkyl-, $-CO$-Phenyl-,

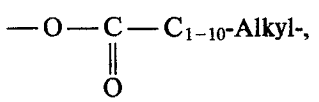

$-COO-C_{1-10}$-Alkyl-, $-COO$-Phenyl-, Phenyl- oder Naphthylgruppen, die ihrerseits durch Halogenatome, $C_{1-10}$-Alkyl-, $C_{1-10}$-Alkoxy-, Di-($C_{1-10}$-alkyl)amino-, Nitro-, Cyano-, Trifluormethyl-, $-CO-C_{1-10}$-Alkyl-, $-CO$-Phenyl-, $-COO-C_{1-10}$-Alkyl- oder $-COO$-Phenylgruppen substituiert sein können. Phenyl- und Naphthylsubstituenten an Gruppen Z oder $Z_1$ sind bevorzugt monosubstituiert oder unsubstituiert. Alkylgruppen R sowie Alkyl- und Alkoxygruppen in den obenerwähnten Substituenten können geradkettig oder verzweigt sein, wobei Alkyl- und Alkoxysubstituenten an Gruppen Z oder $Z_1$ bevorzugt 1 bis 8 und insbesondere 1 bis 4 C-Atome aufweisen. Halogensubstituenten sind z. B. Fluor, Chlor oder Brom. Als Beispiele definitionsgemäßer Gruppen R bezw. Substituenten an Gruppen Z oder $Z_1$ seien erwähnt: die Methyl-, Äthyl-, n-Propyl-, Isopropyl-, n-, sek.- und tert.-Butyl-, n-Pentyl-, 2-Pentyl-, n-Hexyl-, n-Heptyl-, n-Octyl- und n-Decylgruppe; die Methoxy-, Äthoxy-, n-Propoxy-, n-Butoxy-, n-Hexyloxy- und n-Decyloxygruppe; die N,N-Dimethylamino-, N,N-Diäthylamino-, N,N-Di-n-propylamino-, N,N-Di-n-butylamino-, N,N-Di-n-hexylamino-, N,N-Di-n-octylamino-, N-Methyl-N-äthylamino-, N-Methyl-N-n-propylamino-, N-Äthyl-N-n-hexylamino- und N-Äthyl-N-n-butylaminogruppe; die Methyl- und Äthylsulfonylgruppe; die Acetyl-, Propionyl-, Butyryl-, Valeroyl- und Octanoylgruppe; die Carbonsäuremethyl-, -äthyl-, -n-propyl-, -isopropyl-, -n-butyl-, -n-pentyl-, -n-hexyl-, -n-heptyl- und -n-decylestergruppe; $-CH{=}CHCOOCH_3$ und $-CH{=}CHCOOC_2H_5$.

Alkylgruppen R sind bevorzugt geradkettig und weisen ein oder zwei C-Atome auf. X in Formel II stellt vorzugsweise Chlor dar.

Als Verbindungen der Formel II und III kommen insbesondere in Betracht:

1. Verbindungen der Formeln IIa und IIIa

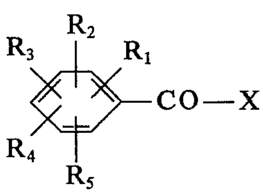

(IIa)

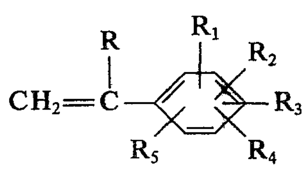

(IIIa)

worin X Chlor oder Brom, R Methyl und besonders Wasserstoff, $R_1$ Wasserstoff, Cl, Br, F, J, Formyl, $-CH(OCH_3)_2$, $-CH(OC_2H_5)_2$,

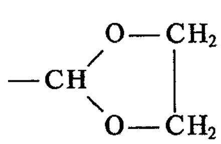

$-CH{=}CH-CN$, $-CH{=}CHCOOCH_3$, $-CH{=}CHCOOC_2H_5$, $C_{1-4}$-Alkyl, $C_{1-4}$-Alkoxy, Phenoxy, Di($C_{1-2}$-alkyl)amino, $-NO_2$, $-CN$, $-CF_3$, $C_{1-4}$-Alkylsulfonyl, Benzyl, $-CO-C_{1-4}$-Alkyl, $-CO$-Phenyl, $-OCO-C_{1-4}$-Alkyl, $-COO-C_{1-4}$-Alkyl, $-COO$-Phenyl, Phenyl, Chlorphenyl, Bromphenyl, Methylphenyl, Methoxyphenyl, 1- oder 2-Naphthyl, $R_2$ und $R_3$ unabhängig voneinander Wasserstoff, Cl, Br, F, $-NO_2$, $C_{1-4}$-Alkyl oder $C_{1-4}$-Alkoxy, besonders Methyl oder Methoxy, $R_4$ oder $R_5$ Wasserstoff oder, wenn $R_1$, $R_2$ und $R_3$ je Chlor, Brom, Fluor oder Methyl sind, ebenfalls je Chlor, Brom, Fluor oder Methyl bedeuten. Bevorzugt sind Verbindungen der Formel IIIa und vor allem solche der Formel IIa, worin X Chlor, R Methyl und besonders Wasserstoff, $R_1$ Wasserstoff,

Cl, Br, F, J, $-CH=CHCN$, $-CH=CHCOOCH_3$, $-CH=CHCOOC_2H_5$, $C_{1-4}$-Alkyl, besonders Methyl oder Äthyl, Methoxy, N,N-Dimethylamino, $-NO_2$, $-CN$, Formyl, Methylsulfonyl oder Phenyl, $R_2$ Wasserstoff, Cl, Br, Methyl, Äthyl, Methoxy oder Nitro, $R_3$ Wasserstoff, Cl, Br, Methyl, Äthyl oder Methoxy und $R_4$ und $R_5$ je Wasserstoff bedeuten.

2. Verbindungen der Formel IIb

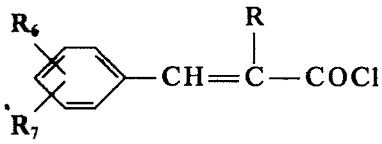

(IIb)

worin R Methyl und insbesondere Wasserstoff, $R_6$ Wasserstoff, Cl, Br, F, $-NO_2$, $-CN$, $-SO_2CH_3$, Methyl, Äthyl, Methoxy, Äthoxy, $-CHO$ oder $-CH(OCH_3)_2$ und $R_7$ Wasserstoff, Cl, Br, F, $-NO_2$, Methyl, Äthyl, Methoxy oder Äthoxy darstellen. Bevorzugte Verbindungen der Formel IIb sind solche, worin R Methyl und insbesondere Wasserstoff, $R_6$ Wasserstoff, Methyl, Methoxy, Cl, Br, F, $-NO_2$ oder $-CHO$ und $R_7$ Wasserstoff bedeuten.

3. Verbindungen der Formel IIc

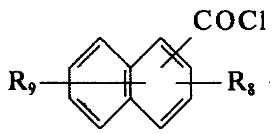

(IIc)

worin die Gruppe $-COCl$ in 1- oder 2-Stellung ist, $R_8$ und $R_9$ an denselben Ring oder an verschiedene Ringe gebunden sein können, $R_8$ Wasserstoff, Cl, Fr, F, Methyl, Äthyl, Methoxy, Äthoxy, $-CHO$, $-COCH_3$, $-SO_2CH_3$, $-CN$, $-NO_2$ oder $-CH(OCH_3)_2$ und $R_9$ Wasserstoff, Cl, Br, F, Methyl, Methoxy oder $-NO_2$ bedeuten. Als Verbindungen der Formel IIc werden solche bevorzugt, worin $R_8$ Methyl und insbesondere Wasserstoff und $R_9$ Wasserstoff darstellen.

4. Verbindungen der Formel IId

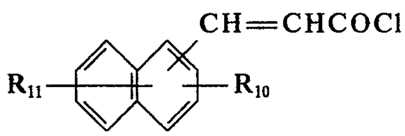

(IId)

worin die Gruppe $-CH=CHCOCl$ in 1- oder 2-Stellung gebunden ist, $R_{10}$ und $R_{11}$ an denselben Ring oder an verschiedene Ringe gebunden sein können, $R_{10}$ Wasserstoff, Cl, Br, F, Methyl, Methoxy, $-NO_2$, $-CHO$, $-CN$, $-SO_2CH_3$ oder $-CH(OCH_3)_2$ und $R_{11}$ Wasserstoff, Cl, Br, F, Methyl, Methoxy oder $-NO_2$ bedeuten. Bevorzugte Verbindungen der Formel IId sind solche, worin $R_{10}$ Wasserstoff oder Methyl und $R_{11}$ Wasserstoff darstellen.

5. Verbindungen der Formel IIe

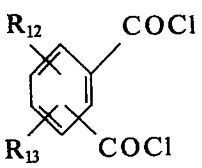

(IIe)

worin $R_{12}$ Wasserstoff, $-CO$-Phenyl, Cl, Br, F, $-CN$, $-CHO$, $-NO_2$ oder Methyl und $R_{13}$ Wasserstoff, Cl, Br, F oder Methyl bedeuten. Als Verbindungen der Formel IIe werden Iso- und Terephthalsäuredichlorid, die gegebenenfalls durch eine Methyl- oder $NO_2$-Gruppe substituiert sind und vor allem das unsubstituierte Iso- oder Terephthalsäuredichlorid, bevorzugt.

6. Verbindungen der Formel IIf

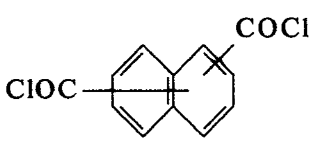

(IIf)

wobei die $-COCl$-Gruppen an denselben Ring oder an verschiedene Ringe gebunden sein können. Als Verbindungen der Formel IIf werden 1,4- und 2,6-Naphthalindicarbonsäuredichlorid bevorzugt.

7. Verbindungen der Formel IIg

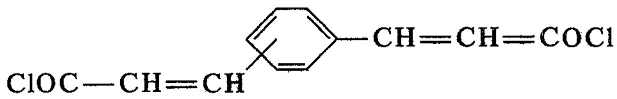

(IIg)

wobei die $-CH=CH-COCl$-Gruppen bevorzugt in 1,3- oder 1,4-Stellung gebunden sind.

8. Verbindungen der Formel IIh

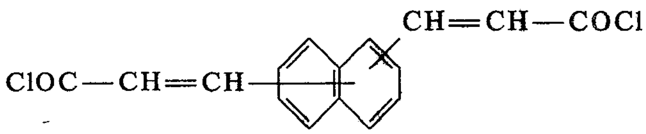

(IIh)

wobei die $-CH=CH-COCl$-Gruppen an denselben Ring oder an verschiedene Ringe und bevorzugt in 1,4- oder 2,6-Stellung gebunden sind.

9. Die Verbindung der Formel IIi

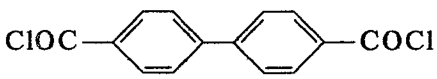

(IIi)

R stellt vorzugsweise Methyl und insbesondere Wasserstoff dar. Als Verbindungen der Formel I mit $p=2$ werden symmetrische Verbindungen bevorzugt, d. h. solche, worin R und $Z_1$ in den beiden Gruppen $-CH=C(R)-Z_1$ je dieselbe Bedeutung haben.

Besonders bevorzugt als Säurehalogenide sind Verbindungen der Formel IIa, worin X Chlor, $R_1$ Wasserstoff, Formyl, Methyl, Methoxy, Cyano, Nitro, Cl, Br, F, J, $-CH=CHCN$, $-CH=CHCOOCH_3$, $-CH=CHCOOC_2H_5$ oder Phenyl, $R_2$ Wasserstoff, Methyl, Methoxy, Cl oder Br, $R_3$ Wasserstoff oder Methoxy und $R_4$ und $R_5$ je Wasserstoff bedeuten; Verbindungen der Formel IIb, worin R Methyl und vor allem Wasserstoff, $R_6$ Wasserstoff, Cl oder Nitro und $R_7$ Wasserstoff darstellen; Verbindungen der Formel IIc, worin $R_8$ Methyl und vor allem Wasserstoff und $R_9$ Wasserstoff bedeuten; unsubstituiertes Iso- oder Terephthalsäuredichlorid; 1,4- oder 2,6-Naphthalindicarbonsäuredichlorid; Verbindungen der Formel IIg, worin sich die $-CH=CH-COCl$-Gruppen in 1,3- oder 1,4-Stellung befinden, sowie das 4,4'-Diphenyldicarbonsäuredichlorid. Besonders bevorzugt verwendet man 4-Brombenzoylchlorid oder Benzoylchlorid.

Bevorzugte Verbindungen der Formel III sind solche, worin R Methyl und insbesondere Wasserstoff und $Z_1$ Chlorphenyl, Bromphenyl, Zimtsäurenitril, Methylphenyl, Methoxyphenyl, Napthyl und besonders Phenyl darstellen. 4-Bromstyrol und Styrol sind ganz besonders bevorzugt als Verbindungen der Formel III.

Die Katalysatoren sowie die Verbindungen der Formeln II und III sind bekannt oder können nach an sich bekannten Methoden hergestellt werden. Bezüglich der Herstellung von Verbindungen der Formel II vgl. z. B. »Organikum«, 387–388, VEB Deutscher Verlag der Wissenschaften, Berlin 1964. Die Verbindungen der Formeln II und III werden in mindestens stöchiometrischer Menge eingesetzt. Bevorzugt verwendet man einen Überschuß an Verbindungen der Formel III, z. B. bis ca. 1,5 Mol Verbindung der Formel III pro Säurehalogenidgruppe.

Als definitionsgemäße Palladiumverbindungen können — neben Palladium-Metall — z. B. Verbindungen der Formel IV

$$M^y [PdL_n]^x \qquad (IV)$$

eingesetzt werden, worin n eine ganze Zahl von 2 bis 4, x $2^+$ bis $2^-$, $y=-(x)$, M, bei x ungleich 0, ein Gegenion und die L gleiche oder verschiedene phosphorfreie Liganden darstellen, wie z. B.

Cl, Br, J, $-CN$, $-NO_3$, $C_{1-12}$-Alkyl$-COO$, $CH_3COCHCOCH_3$, $NH_3$, 2,2'-Bipyridyl,

o-Phenanthrolin, $OS(CH_3)_2$ oder $-NC$-Phenyl.

Geeignete Verbindungen der Formel IV sind beispielsweise

$PdCl_2$, $PdBr_2$, $Pd(CN)_2$, $Pd(NO_3)_2$, $Pd(O_2C-C_{1-12}\text{-Alkyl})_2$, besonders $Pd(OOCCH_3)_2$,

$Pd(CH_3COCHCOCH_3)_2$, $[Pd(NH_3)_4]Cl_2$, $[PdCl_4]Na_2$, $Pd(OOCCH_3)_2$ (2,2'-Bipyridyl), $Pd(OOCCH_3)_2$-(o-Phenanthrolin), $PdCl_2[OS(CH_3)_2]_2$ und $PdCl_2$(NC-Phenyl)$_2$.

Außer den obengenannten Verbindungen können auch Palladiumverbindungen anderer Oxidationsstufen eingesetzt werden, z. B. Bis-(Dibenzylidenaceton)Palladium(0) und Bis-(Isonitril)Palladium(0)-Verbindungen. Als Beispiele solcher Isonitrile seien genannt:

Bis-(Cyclohexylisonitril)Palladium(0), Bis-(Isopropylisonitril)Palladium(0),
Bis-(tert.-Butylisonitril)Palladium(0), Bis-(p-Tolylisonitril)Palladium(0),
Bis-(Phenylisonitril)Palladium(0) und Bis-(p-Methoxyphenylisonitril)Palladium(0).

Davon sind

Bis-(Dibenzylidenaceton)Palladium(0), Bis-(Cyclohexylisonitril)Palladium(0) und
Bis-(Isopropylisonitril)Palladium(0)

bevorzugt.

Bevorzugt verwendet man als Katalysatoren

$PdCl_2$, $PdBr_2$, $Pd(OOCCH_3)_2$, $Pd(CH_3COCHCOCH_3)_2$, $Pd(OOCCH_3)_2$ (2,2'-Bipyridyl), $PdCl_2$(NC-Phenyl)$_2$, Bis-(Dibenzylidenaceton) Palladium (0) und Bis-(Cyclohexylisonitril)-Palladium (0).

Ganz besonders bevorzugt sind

$PdCl_2$, Palladiumacetat und Bis-(Dibenzylidenaceton) Palladium (0).

Die Katalysatoren werden im allgemeinen in einer Menge von 0,0001 bis 20 Mol-%, bevorzugt 0,001 bis 3 Mol-%, bezogen auf die Verbindung der Formel II, eingesetzt.

Als Basen können im erfindungsgemäßen Verfahren sowohl anorganische als auch organische Verbindungen, die im Reaktionsmedium ausreichend löslich sind, verwendet werden. Geeignete Basen sind beispielsweise Verbindungen der Formeln V bis VII

$$(Z')^{n\oplus}\,(^{\ominus}OOCQ')_n \qquad (V)$$

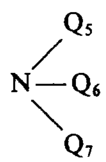

(VI)

oder

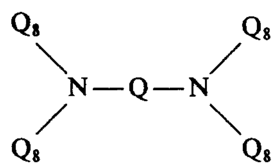

(VII)

sowie cyclische tertiäre Amine, z. B. N-Methyl- oder N-Äthylpiperidin, 1,2,2,6,6-Pentamethylpiperidin, 4-Oxo-1,2,2,6,6-pentamethylpiperidin, 1,4-Diazabicyclo[2.2.2]octan (DABCO), N-Alkylmorpholine und N-Alkylpyrrolidine, wie N-Methyl- und N-Äthylmorpholin, N-Methyl- und N-Äthylpyrrolidin, oder N,N'-Dialkylpiperazine, wie N,N'-Dimethylpiperazin.

In den obigen Formeln bedeuten:

n die Zahl 1 oder 2,
Q' Phenyl oder $C_{1-17}$-Alkyl
Z' ein Alkalimetallkation, ein Erdalkalimetallkation oder

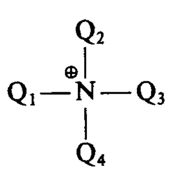

Q geradkettiges oder verzweigtes Alkylen mit 2–6 C-Atomen,
$Q_1$ Wasserstoff, $C_{1-12}$-Alkyl, Cyclopentyl, Cyclohexyl, Benzyl oder Phenyl,
$Q_2$, $Q_3$ und $Q_4$ gleiches oder verschiedenes $C_{1-12}$-Alkyl,
$Q_5$ $C_{1-12}$-Alkyl, Cyclopentyl, Cyclohexyl, Phenyl oder Benzyl, das auch substituiert sein kann, beispielsweise durch ein Halogenatom, wie Chlor oder Brom, eine Alkyl- oder Alkoxygruppe mit je 1–4 und besonders 1 oder 2 C-Atomen,
$Q_6$ und $Q_7$ gleiches oder verschiedenes $C_{1-12}$-Alkyl und
$Q_8$ Methyl oder Äthyl.

Z' in der Bedeutung eines Alkalimetallkations ist besonders das Natrium- und vor allem das Lithiumkation. Alkylgruppen Q' und $Q_1$ bis $Q_7$ können geradkettig oder verzweigt sein. Stellen $Q_5$ bis $Q_7$ Alkylgruppen dar, so weisen diese mit Vorteil zusammen mindestens 8 C-Atome auf, während Alkylgruppen $Q_1$ bis $Q_4$ bevorzugt je 1–4 C-Atome aufweisen. Beispiele von Verbindungen der Formeln V bis VII sind: das Lithiumacetat, -butyrat und -stearat, das Barium- und Calciumacetat, das Kalium-, Calcium- und Natriumstearat, das Lithium- und Natriumbenzoat, sowie die entsprechenden Trimethyl-, Tetramethyl-, Tetraäthyl- und Tetra-n-butylammoniumsalze; Triäthylamin, Tri-n-butylamin, Tri-(2-äthylhexylamin), Tri-n-octylamin und Tri-n-dodecylamin; N-Benzyldialkylamine, wie N-Benzyldimethylamin, N-Benzyldiäthylamin, N-(4-Chlorbenzyl)-dimethylamin und N-(3-Methyl- oder 3-Methoxybenzyl)-dimethylamin; N,N,N',N'-Tetramethyl- und N,N,N',N'-Tetraäthyl-äthylendiamin, N,N,N',N'-Tetramethyl-1,3-diaminopropan und N,N,N',N'-Tetramethyl-1,6-diaminohexan.

Bevorzugt verwendet man als Basen tertiäre Amine der vorerwähnten Art, vor allem N-Äthylmorpholin oder Verbindungen der Formel VI, worin $Q_5$ 4-Chlorbenzyl, 3-Methylbenzyl oder 3-Methoxybenzyl und insbesondere Benzyl und $Q_6$ und $Q_7$ je Alkyl mit 1–4 C-Atomen, vor allem 1 oder 2 C-Atomen, darstellen, oder worin $Q_5$, $Q_6$ und $Q_7$ je Alkyl mit 3–12 C-Atomen darstellen. Besonders bevorzugt sind N-Benzyldimethylamin, N-Äthylmorpholin und Tri-n-butylamin.

Die Reaktionstemperaturen für die erfindungsgemäße Umsetzung liegen zweckmäßig zwischen 0 und 200°C, vorzugsweise zwischen 90 und 150°C. Sind die Säurehalogenide der Formel II flüssig, so kann die Umsetzung ohne Zusatz eines Lösungsmittels durchgeführt werden. Bevorzugt wird die Reaktion jedoch in einem gegenüber den Reaktionspartnern inerten organischen Lösungsmittel durchgeführt. Geeignete inerte organische Lösungsmittel sind z. B. gegebenenfalls chlorierte aliphatische, cycloaliphatische oder aromatische Kohlenwasserstoffe, wie n-Pentan, n-Heptan, n-Octan, Cyclopentan, Cyclohexan, Benzol, Toluol, Xylole und Chlorbenzol; aromatische, aliphatische und cyclische Äther, wie Anisol, Diäthyläther, Di-isopropyläther, Tetrahydrofuran und Dioxan; Nitrile, besonders Benzonitril und Alkylnitrile mit 2 bis 5 C-Atomen, wie Acetonitril, Propionitril und Butyronitril; 3-Methoxypropionitril und 3-Äthoxypropionitril; N,N-Dialkylamide von aliphatischen Monocarbonsäuren mit 1 bis 3 C-Atomen im Säureteil, wie N,N-Dimethylformamid und N,N-Dimethylacetamid; tertiäre Alkohole mit 4 bis 8 C-Atomen, vor allem tert.-Butanol; aliphatische und cycloaliphatische Ketone, wie Aceton, Diäthylketon, Methylisopropylketon, Cyclopentanon und Cyclohexanon; Ester, wie Ester der Kohlensäure, z. B. Diäthylcarbonat, und Alkyl- oder Alkoxyalkylester von aliphatischen Monocarbonsäuren mit insgesamt 2 bis 8 C-Atomen, wie Essigsäuremethyl-, -äthyl-, -n-butyl- und -isobutylester, Buttersäureäthyl- und -n-butylester sowie 1-Acetoxy-2-methoxyäthan. Bevorzugte Lösungsmittel sind Nitrile, Ketone, Ester, cyclische Äther und aromatische Kohlenwasserstoffe der obenerwähnten Art. Für die Umsetzung in Gegenwart anorganischer Basen eignen sich vor allem polare Lösungsmittel, wie Nitrile, Ketone und Ester. Ganz besonders bevorzugt wird die Umsetzung in Gegenwart einer organischen Base und eines aromatischen Äthers oder Kohlenwasserstoffs, vor allem Anisol, Xylolen oder Toluol, vorgenommen.

Beim erfindungsgemäßen Verfahren läßt sich der Reaktionsablauf leicht anhand der CO-Entwicklung verfolgen, beispielsweise mittels eines Blasenzählers. Bei Reaktionsprodukten, die im Reaktionsgemisch beschränkt löslich sind, empfiehlt es sich, die Reaktion nach Beendigung der CO-Entwicklung zu unterbrechen und das Reaktionsprodukt direkt aufzuarbeiten.

Die erfindungsgemäß herstellbaren Verbindungen und deren Verwendungen sind zum großen Teil bekannt; sie können beispielsweise zum Teil direkt als optische Aufheller oder Scintillatoren eingesetzt werden. Derartige optische Aufheller sind z. B. in den britischen Patentschriften 1 247 934 und 1 445 231 beschrieben. Als Scintillatoren eignen sich beispielsweise Verbindungen der in der britischen Patentanmedlung 2 015 021 beschriebenen Art [vgl. auch A. Dyer, »An Introduction to Liquid Scintillation Counting«, Heyden Publ., London–New-York, 1947, S. 16]. Die erfindungsgemäß hergestellten Verbindungen können ferner auf an sich bekannte Weise, gegebenenfalls unter Einführung von geeigneten funktionellen Gruppen, wie Aminogruppen, und/oder durch Sulfonierung der aromatischen Reste Z und $Z_1$, in Farbstoffe oder optische Aufheller übergeführt werden [vgl. z. B. Encyclopedia of Chemical Technology, 2. Auflage, Bd. 19, S. 1 bis 16]. Stilbene und Stilbenderivate finden auch Anwendung als Klebstoffadditive, Insektizide oder Lichtstabilisatoren; vgl. z. B. Chemical Abstracts 78, 39 352; 84, 137 386 und 85, 22 416.

## Beispiel 1

7,05 g (0,05 Mol) Benzoylchlorid werden zusammen mit 6,5 g (0,0625 Mol) Styrol, 8,65 g (0,05 Mol) Tri-n-butylamin, 0,1122 g (0,0005 Mol) Palladiumacetat und 100 ml p-Xylol in einen 250-ml-Kolben eingefüllt und unter Rühren auf 120° C erwärmt. Es ist eine leichte Gasentwicklung zu beobachten. Nach einer Rührzeit von 2 Stunden bei 120° C wird abgekühlt, mit 2 x 25-ml-Portionen an 2 n Salzsäure ausgeschüttelt, und die organische Phase wird mit Magnesiumsulfat getrocknet. Das p-Xylol wird abdestilliert, und der Rückstand wird aus Äthanol umkristallisiert. Man erhält 8,0 g (0,0445 Mol) Stilben als farblose, kristalline Verbindung, entsprechend einer Ausbeute von 89% d. Th.; Fp. 125° C.

Analyse für $C_{14}H_{12}$ (Molgewicht 180):
berechnet: C 93,29%, H 6,71%;
gefunden: C 93,60%, H 6,63%.

## Beispiel 2

Es wird wie in Beispiel 1 beschrieben vorgegangen, jedoch unter Verwendung von 8,43 g (0,05 Mol) 4-Formylbenzoylchlorid, 6,5 g (0,0625 Mol) Styrol, 17,5 g (0,05 Mol) Tri-n-octylamin, 100 ml Dioxan als Lösungsmittel und 0,287 g (0,0005 Mol) Bis-(Dibenzylidenaceton)palladium(0). Nach einer Reaktionszeit von 2 Stunden bei 120° C erhält man 5,8 g (0,0279 Mol) 4-Formylstilben als gelbliche Kristalle, entsprechend einer Ausbeute von 56% d. Th.; Fp. 112 – 113° C.

Analyse für $C_{15}H_{12}O$ (Molgewicht 208):
berechnet: C 86,51%, H 5,81%, O 7,68%;
gefunden: C 85,99%, H 6,08%, O 7,33%.

## Beispiel 3

Es wird wie in Beispiel 1 beschrieben vorgegangen, jedoch unter Verwendung von 17,5 g (0,1 Mol) 4-Chlorbenzoylchlorid, 13,02 g (0,125 Mol) Styrol, 35,37 g (0,1 Mol) Tri-2-äthylhexylamin und 0,1773 g (0,01 Mol) Palladiumchlorid. Nach einer Reaktionszeit von 4 Stunden bei 120° C erhält man 9,3 g (0,0433 Mol) 4-Chlorstilben in Form von weißen Blättchen, entsprechend einer Ausbeute von 43,3% d. Th; Fp. 129 – 130° C.

Analyse für $C_{14}H_{11}Cl$ (Molgewicht 214,5):
berechnet: C 78,32%, H 5,16%;
gefunden: C 78,07%, H 5,16%.

## Beispiel 4

Es wird wie in Beispiel 1 beschrieben vorgegangen, jedoch unter Verwendung von 20,3 g (0,1 Mol) Terephthalsäuredichlorid, 26,04 g (0,25 Mol) Styrol, 53,9 g (0,25 Mol) Tri-n-hexylamin und 0,5329 g (0,002 Mol) Palladiumnitratdihydrat. Nach einer Reaktionszeit von 3 Stunden bei 120° C erhält man 8,5 g (0,0301 Mol) 4,4'-Distyrylbenzol in Form von gründlich gelben Blättchen, entsprechend einer Ausbeute von 30,1% d. Th.; Fp. 267° C.

Analyse für $C_{22}H_{18}$ (Molgewicht 282):
berechnet: C 93,58%, H 6,43%;
gefunden: C 93,58%, H 6,44%.

## Beispiel 5

Es wird wie in Beispiel 1 beschrieben vorgegangen, jedoch unter Verwendung von 13,95 g (0,05 Mol) 4,4'-Biphenyldicarbonsäuredichlorid, 13,0 g (0,125 Mol) Styrol, 23,13 g (0,125 Mol) Tri-n-butylamin und 0,224 g (0,001 Mol) Palladiumacetat. Nach einer Reaktionszeit von 3 Stunden in 100 ml Propionitril als Lösungsmittel bei 100° C erhält man 5,1 g (0,0143 Mol) 4,4'-Distyryl-biphenyl, entsprechend einer Ausbeute von 28,6% d. Th.; Fp. 323 – 326° C.

Analyse für $C_{28}H_{22}$ (Molgewicht 358):
berechnet: C 93,81%, H 6,19%;
gefunden: C 93,39%, H 6,08%.

## Beispiel 6

Es wird wie in Beispiel 1 beschrieben vorgegangen, jedoch unter Verwendung von 16,7 g (0,1 Mol) Zimtsäurechlorid, 13,0 g (0,125 Mol) Styrol, 23,3 g (0,125 Mol) Tri-n-butylamin, 100 ml Chlorbenzol und 0,225 g (0,001 Mol Palladiumacetat. Nach einer Reaktionszeit von 5 Stunden bei einer Badtemperatur von 140° C erhält man 5,7 g (0,0277 Mol) 1,4-Diphenyl-1,3-butadien, entsprechend einer Ausbeute von 27,7% d. Th.; Fp. 150—152° C.

## Beispiel 7

Es wird wie in Beispiel 1 beschrieben vorgegangen, jedoch unter Verwendung von 19,1 g (0,1 Mol) 2-Naphthoylchlorid, 13,1 g (0,125 Mol) Styrol, 23,2 g (0,125 Mol) Tri-n-butylamin und 0,225 g (0,001 Mol) Palladiumacetat. Nach einer Reaktionszeit von 2 Stunden bei 130° C in 100 ml Buttersäureäthylester erhält man 10,2 g (0,0445 Mol) 2-Styrylnaphthalin, entsprechend einer Ausbeute von 44,5% d. Th., Fp. 143—145° C.

## Beispiel 8

Es wird wie in Beispiel 1 beschrieben vorgegangen, jedoch unter Verwendung von 15,5 g (0,1 Mol) o-Toluylsäurechlorid, 13 g (0,125 Mol) Styrol, 23,2 g Tri-n-butylamin und 0,225 g Palladiumacetat. Nach einer Reaktionszeit von 4 Stunden bei 120° C in 100 ml Cyclohexanon als Lösungsmittel erhält man 10,8 g (0,0556 Mol) 2-Methylstilben, entsprechend einer Ausbeute von 55,6% d. Th.; Fp. 92—93° C.

## Beispiel 9

Es wird wie in Beispiel 1 beschrieben vorgegangen, jedoch unter Verwendung von 17,1 g (0,1 Mol) 2-Methoxybenzylchlorid, 13 g (0,125 Mol) Styrol, 23,2 g Tri-n-butylamin und 0,225 g (0,001 Mol) Palladiumacetat. Nach einer Reaktionszeit von 3 Stunden bei einer Badtemperatur von 140° C in 100 ml Diäthylcarbonat als Lösungsmittel erhält man 10,3 g (0,049 Mol) 2-Methoxystilben, entsprechend einer Ausbeute von 49% d. Th.; Fp. 69—70° C.

## Beispiel 10

Es wird wie in Beispiel 1 beschrieben vorgegangen, jedoch unter Verwendung von 26,65 g (0,1 Mol) 3-Jodbenzoylchlorid, 13 g (0,125 Mol) Styrol, 12,93 g (0,1 Mol) Äthyl-diisopropylamin und 0,575 g (0,001 Mol) Bis-(Dibenzylidenaceton)-palladium(0). Nach einer Reaktionszeit von 3 Stunden bei Rückflußtemperatur in 150 ml Propionitril erhält man 3,05 g (0,01 Mol) 3-Jodstilben, entsprechend einer Ausbeute von 10% d. Th.; Fp. 96° C.

Analyse für $C_{14}H_{11}J$ (Molgewicht 216):
berechnet: C 54,93%, H 3,62%, J 41,49%;
gefunden: C 55,35%, H 3,70%, J 41,22%.

## Beispiel 11

Es wird wie in Beispiel 1 beschrieben vorgegangen, jedoch unter Verwendung von 15,46 g (0,1 Mol) p-Toluylsäurechlorid, 13,02 g (0,125 Mol) Styrol, 14,3 g (0,1 Mol) Tri-n-propylamin und 0,177 g (0,001 Mol) Palladiumchlorid. Nach einer Reaktionszeit von 3 Stunden bei 130° C in 50 ml Chlorbenzol als Lösungsmittel erhält man 5,8 g (0,03 Mol) 4-Methylstilben, entsprechend einer Ausbeute von 30% d. Th.; Fp. 117° C.

Analyse für $C_{15}H_{14}$ (Molgewicht 194):
berechnet: C 92,74%, H 7,26%;
gefunden: C 92,93%, H 7,26%.

## Beispiel 12

Es wird wie in Beispiel 1 beschrieben vorgegangen, jedoch unter Verwendung von 16,55 g (0,1 Mol) 4-Cyanbenzoylchlorid, 13,02 g (0,125 Mol) Styrol, 35,37 g (0,1 Mol) Tri-n-octylamin, 50 ml

Buttersäureäthylester als Lösungsmittel und 0,0575 g (0,0001 Mol) Bis-(Dibenzylidenaceton)-palladium(0). Nach einer Reaktionszeit von 2 Stunden bei 100° C erhält man 5,5 g (0,0268 Mol) 4-Cyanstilben, entsprechend einer Ausbeute von 26,8% d. Th.; Fp. 119° C.

Analyse für $C_{15}H_{11}N$ (Molgewicht 205):
berechnet: C 87,77%, H 5,40%, N 6,82%;
gefunden: C 87,55%, H 5,62%, N 6,73%.

Beispiel 13

Es wird wie in Beispiel 1 beschrieben vorgegangen, jedoch unter Verwendung von 23,07 g (0,1 Mol) 3,4,5-Trimethoxybenzoylchlorid, 13,02 g (0,125 Mol) Styrol, 12,93 g (0,125 Mol) Äthyldiisopropylamin und 0,3046 g (0,001 Mol) Bis-(Acetylacetonato)Palladium(II). Nach einer Reaktionszeit von 3 Stunden bei 130° C in 50 ml Cyclohexanon als Lösungsmittel erhält man 4,7 g (0,0175 Mol) 3,4,5-Trimethoxystilben in Form von weißen Nadeln, entsprechend einer Ausbeute von 17,5% d. Th.; Fp. 108° C.

Analyse für $C_{17}H_{18}O_3$ (Molgewicht 270):
berechnet: C 75,53%, H 6,71%;
gefunden: C 75,54%, H 6,61%.

Beispiel 14

0,112 mg (0,5 mMol) Palladiumacetat, 10,97 g (50 mMol) 4-Brombenzoylchlorid, 10,17 g (50 mMol) 4-Bromstyrol und 6,76 g (50 mMol) N-Benzyldimethylamin in 10 ml o-Xylol werden während 1 Std. bei 130° C gerührt. Nach dem Abfiltrieren vom ausgefallenen Aminsalz wird das Rohprodukt zweimal aus Toluol umkristallisiert. Man erhält 7,8 g (46% d. Th.) 4,4'-Dibromstilben in Form weißer Blättchen; Fp. 213,3° C.

Analyse für $C_{14}H_{10}Br_2$:
berechnet: C 49,75%, H 2,98%;
gefunden: C 49,79%, H 3,07%.

Beispiel 15

0,448 g (2 mMol) Palladiumacetat, 28,11 g (0,2 Mol) Benzoylchlorid, 23,64 g (0,2 Mol) α-Methylstyrol und 27,04 g (0,2 Mol) N-Benzyldimethylamin in 400 ml p-Xylol werden während 11 Std. bei 130° C gerührt. Das Gemisch wird mit 400 ml 2N HCl und 400 ml 2N NaOH ausgeschüttelt und über Magnesiumsulfat getrocknet. Das Rohprodukt wird auf Kieselgel in Methylenchlorid chromatographiert und anschließend aus n-Pentan umkristallisiert. Man erhält 1,3 g (3% d. Th.) α-Methylstilben als farblose Kristalle vom Fp. 79,0° C.

Analyse für $C_{15}H_{14}$:
berechnet: C 92,74%, H 7,27%;
gefunden: C 92,65%, H 7,17%.

Beispiel 16

Es wird wie in Beispiel 14 beschrieben verfahren, jedoch unter Verwendung von 5,91 g (50 mMol) 4-Methylstyrol. Das Gemisch wird eine Stunde bei 130° C gerührt. Das Rohprodukt wird aus Toluol/Cyclohexan umkristallisiert. Man erhält 7,4 g (54% d. Th.) 4-Brom-4'-methylsilben als weiße Kristalle vom Fp. 214,6° C.

Analyse für $C_{15}H_{13}Br$:
berechnet: C 65,95%, H 4,80%;
gefunden: C 65,72%, H 4,71%.

Beispiel 17

Es wird wie in Beispiel 14 beschrieben vorgegangen, jedoch unter Verwendung von 9,64 g (50 mMol) 1-Vinylnaphthalin. Das Gemisch wird 3 1/2 Stunden bei 130° C gerührt. Das Rohprodukt wird in Toluol

auf Kieselgel chromatographiert und dann einmal aus n-Hexan und einmal aus n-Hexan/n-Pentan umkristallisiert. Man erhält 3,0 g (19% d. Th.) 1-(4-Bromstyryl)-naphthalin als hellgelbe Kristalle vom Fp. 105,6° C.

Analyse für $C_{18}H_{13}Br$:
berechnet: C 69,92%, H 4,24%;
gefunden: C 69,99%, H 4,24%.

Beispiel 18

0,448 g (2 mMOl) Palladiumacetat, 43,88 g (0,2 Mol) 4-Brombenzoylchlorid, 26 g (0,25 Mol) Styrol und 37,06 g (0,2 Mol) Tri-n-butylamin werden in 200 ml p-Xylol während 4 Std. bei 120° C gerührt. Nach dem Ausschütteln des Reaktionsgemisches mit 2N HCl und 2N NaOH und dem Trocknen über Magnesiumsulfat wird das Rohprodukt in Toluol auf Kieselgel chromatographiert und aus n-Hexan umkristallisiert. Man erhält 26,43 g (51% d. Th.) 4-Bromstilben als hellgelbe Kristalle vom Fp. 137,7° C.

Analyse für $C_{14}H_{11}Br$:
berechnet: C 64,89%, H 4,28%;
gefunden: C 65,20%, H 4,32%.

Beispiel 19

Es wird wie in Beispiel 18 beschrieben vorgegangen, jedoch unter Verwendung von 33,7 g (0,2 Mol) 3,4-Dimethylbenzoylchlorid. Das Gemisch wird 2 Std. bei 120° C gerührt. Das Rohprodukt wird einmal aus Methanol und einmal aus n-Hexan umkristallisiert. Man erhält 15,8 g (38% d. Th.) 3,4-Dimethylstilben in Form hellgelber Kristalle vom Fp. 76° C.

Analyse für $C_{16}H_{16}$:
berechnet: C 92,26%, H 7,74%;
gefunden: C 92,06%, H 7,69%.

Beispiel 20

Es wird wie in Beispiel 18 beschrieben vorgegangen, jedoch unter Verwendung von 41,89 g (0,2 Mol) 3,4-Dichlorbenzoylchlorid. Das Gemisch wird 1 Std. bei 120° C gerührt. Das Rohprodukt wird im Soxhlet extrahiert und aus n-Pentan umkristallisiert. Man erhält 28,6 g (57% d. Th.) 3,4-Dichlorstilben in Form weißer Blättchen; Fp. 87,7° C.

Analyse für $C_{14}H_{10}Cl_2$:
berechnet: C 67,50%, H 4,50%, Cl 28,46%;
gefunden: C 67,80%, H 4,08%, Cl 28,65%.

Beispiel 21

Es wird wie in Beispiel 18 beschrieben vorgegangen, jedoch unter Verwendung von 39,91 g (0,2 Mol) 2-Methyl-5-nitrobenzoylchlorid. Das Gemisch wird 1,5 Stunden bei 120° C gerührt. Das Rohprodukt wird im Soxhlet extrahiert und aus Cyclohexan/n-Hexan umkristallisiert. Man erhält 20,9 g (44% d. Th.) 2-Methyl-5-nitrostilben als hellgelbe Kristalle vom Fp. 65,5° C.

Analyse für $C_{15}H_{13}NO_2$:
berechnet: C 75,30%, H 5,48%, N 5,86%;
gefunden: C 75,12%, H 5,62%, N 5,87%.

Beispiel 22

Es wird wie in Beispiel 18 beschrieben vorgegangen, jedoch unter Verwendung von 39,91 g (0,2 Mol) 2-Methyl-3-nitrobenzoylchlorid. Das Gemisch wird 30 Minuten bei 120° C gerührt. Das Rohprodukt wird auf Kieselgel in Toluol chromatographiert und dann aus n-Hexan umkristallisiert. Man erhält 28,5 g (60% d. Th.) 2-Methyl-3-nitrostilben als hellgelbe Kristalle vom Fp. 75,9° C.

Analyse für $C_{15}H_{13}NO_2$:
berechnet: C 75,30%, H 5,48%, N 5,86%;
gefunden: C 75,30%, H 5,64%, N 5,88%.

## Beispiel 23

Es wird wie in Beispiel 18 beschrieben vorgegangen, jedoch unter Verwendung von 40,1 g (0,2 Mol) 3,5-Dimethoxybenzoylchlorid. Das Gemisch wird 2,5 Std. bei 120°C gerührt. Das Rohprodukt wird auf Kieselgel in Methylenchlorid chromatographiert und anschließend aus n-Hexan umkristallisiert. Man erhält 27,6 g (58% d. Th.) 3,5-Dimethoxystilben als weiße Kristalle vom Fp. 56,4°C.

Analyse für $C_{16}H_{16}O_2$:
berechnet: C 79,98%, H 6,71%;
gefunden: C 80,6%, H 6,8%.

## Beispiel 24

Es wird wie in Beispiel 18 beschrieben vorgegangen, jedoch unter Verwendung von 43,30 g (0,2 Mol) Biphenyl-4-carbonsäurechlorid. Das Gemisch wird während 3 Std. bei 120°C gerührt. Das Rohprodukt wird abfiltriert und aus Toluol umkristallisiert. Man erhält 28,5 g (56% d. Th.) 4-Phenylstilben als hellgelbe Kristalle vom Fp. 223,7°C..

Analyse für $C_{20}H_{16}$:
berechnet: C 93,71%, H 6,29%;
gefunden: C 93,61%, H 6,24%.

## Beispiel 25

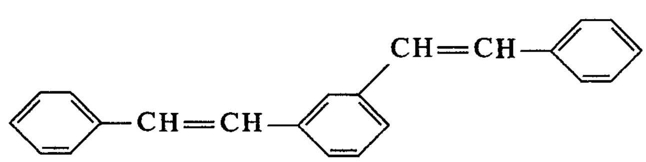

0,896 g (4 mMol) Palladiumacetat, 40,6 g (0,2 Mol) Isophthalsäuredichlorid, 52,0 g (0,5 Mol) Styrol und 74,12 g (0,4 Mol) Tri-n-butylamin in 300 ml p-Xylol werden während 2¾ Std. bei 120°C gerührt. Das Rohprodukt wird im Soxhlet extrahiert und aus n-Hexan/Cyclohexan umkristallisiert. Man erhält 14,0 g (25% d. Th.) der obigen Verbindung in Form weißer Kristalle vom Fp. 173,0°C.

Analyse für $C_{22}H_{18}$:
berechnet: C 93,58%, H 6,45%;
gefunden: C 93,51%, H 6,77%.

## Beispiel 26

0,224 g (1 mMol) Palladiumacetat, 12,65 g (50 mMol) Naphthalin-2,6-dicarbonsäuredichlorid, 13,04 g (125 mMol) Styrol und 18,53 g (100 mMol) Tri-n-butylamin in 50 ml p-Xylol werden während 2 Std. bei 120°C gerührt. Das Rohprodukt wird im Soxhlet extrahiert und aus Tetrahydrofuran umkristallisiert. Man erhält 6,1 g (37% d. Th.) 2,6-Distyrylnaphthalin als hellgelbe Kristalle vom Fp. 294,7°C.

Analyse für $C_{26}H_{20}$:
berechnet: C 93,93%, H 6,07%;
gefunden: C 93,73%, H 6,24%.

## Beispiel 27

Es wird wie in Beispiel 26 beschrieben vorgegangen, jedoch unter Verwendung von 12,65 g (50 mMol) Naphthalin-1,4-dicarbonsäuredichlorid. Das Gemisch wird während 2 Std. bei 120°C gerührt. Das Rohprodukt wird auf Kieselgel in Methylenchlorid chromatographiert und anschließend aus Cyclohexan umkristallisiert. Man erhält 10,6 g (64% d. Th.) 1,4-Distyrylnaphthalin als gelbe Kristalle

vom Fp. 189° C.

Analyse für $C_{26}H_{20}$:
berechnet: C 93,93%, H 6,07%;
gefunden: C 93,40%, H 6,10%.

Beispiel 28

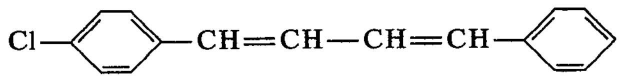

(1-Phenyl-4-(4-chlorphenyl)-but a-1,3-dien)

0,112 g (0,5 mMol) Palladiumacetat, 10,05 g (50 mMol) 4-Chlorzimtsäurechlorid, 6,5 g (62,5 mMol) Styrol und 9,27 g (50 mMol) Tri-n-butylamin in 50 ml p-Xylol werden während 2³/₄ Std. bei 120° C gerührt. Das Rohprodukt wird auf Kieselgel in Methylenchlorid chromatographiert und aus n-Hexan umkristallisiert. Man erhält 0,5 g (4% d. Th.) der obigen Verbindung in Form weißer Kristalle vom Fp. 161,2° C.

Analyse für $C_{16}H_{13}Cl$:
berechnet: C 79,83%, H 5,45%, Cl 14,73%;
gefunden: C 79,63%, H 5,35%, Cl 14,79%.

Beispiel 29

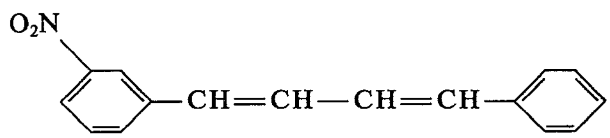

(1-Phenyl-4-(3-nitrophenyl)-buta-1,3-dien)

Man geht wie in Beispiel 28 beschrieben vor, jedoch unter Verwendung von 10,58 g (50 mMol) 3-Nitrozimtsäurechlorid. Das Gemisch wird 3 Std. bei 120° C gerührt. Das Rohprodukt wird auf Kieselgel in Toluol chromatographiert und aus Cyclohexan umkristallisiert. Man erhält 0,8 g (6% d. Th.) der obigen Verbindung als gelbe Kristalle vom Fp. 143,9° C.

Analyse für $C_{16}H_{13}NO_2$:
berechnet: C 76,48%, H 5,22%, N 5,58%;
gefunden: C 76,49%, H 5,19%, N 5,59%.

Beispiel 30

0,095 g (0,25 mMol) Diacetatobipyridylpalladium(II), 3,52 g (25 mMol) Benzoylchlorid, 2,61 g (25 mMol) Styrol und 3,38 g (25 mMol) N-Benzyldimethylamin in 50 ml p-Xylol werden während 100 Minuten bei 130° C gerührt. Nach der Aufarbeitung wie in den vorangehenden Beispielen beschrieben erhält man 2,71 g (52% d. Th.) Stilben.

Beispiel 31

1,12 mg (0,005 mMol) Palladiumacetat, 7,04 g (50 mMol) Benzoylchlorid, 5,22 g (50 mMol) Styrol und 6,76 g (50 mMol) N-Benzyldimethylamin in 100 ml p-Xylol werden während 11,5 Std. bei 130° C gerührt. Man erhält 6,54 g (63% d. Th.) Stilben. Dies entspricht 6300 Mol Stilben pro Mol Katalysator.

Beispiel 32

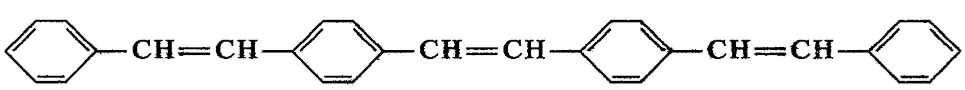

0,1122 g (0,0005 Mol) Palladiumacetat werden in 100 ml o-Xylol gelöst; dann werden der Reihe nach

7,63 g (0,025 Mol) Stilben-4,4'-dicarbonsäuredichlorid, 5,75 ml (0,050 Mol) Styrol und 7,53 ml (0,050 Mol) N-Benzyldimethylamin zugegeben. Nach einer Reaktionszeit von 1,5 Std. bei 130° C und der anschließenden Aufarbeitung erhält man 1,6 g Stilben-4,4'-distyryl, entsprechend einer Ausbeute von 17% d. Th.; Fp. >300° C.

Analyse für $C_{30}H_{24}$:
berechnet: C 93,71%, H 6,29%;
gefunden: C 93,36%, H 6,45%.

Beispiel 33

Unter Verwendung verschiedener Katalysatoren wird nach der in den vorangehenden Beispielen beschriebenen Arbeitsweise trans-Stilben wie folgt hergestellt: Zu 50 ml p-Xylol gibt man unter Argon je 0,25 mMol der unten aufgeführten Katalysatoren, 2,89 ml (25 mMol) Benzoylchlorid, 2,88 ml (25 mMol) Styrol und 3,77 ml (25 mMol) N-Benzyldimethylamin und rührt das Reaktionsgemisch während 1–3 Std. bei 130°C. Nach dem Aufarbeiten erhält man trans-Stilben in den unten angegebenen Ausbeuten:

| Katalysator | Ausbeute |
|---|---|
| 0,08 g (0,25 mMol) Bis-(Cyclohexylisonitril)-Palladium(0), 2 Std. gerührt | 70% d. Th. |
| 0,0833 g (0,25 mMol) $PdCl_2[OS(CH_3)_2]_2$, 2 Std. gerührt | 15% d. Th. |
| 0,0958 g (0,25 mMol) $PdCl_2$(NC-Phenyl)$_2$, 1 Std. gerührt | 74% d. Th. |
| 0,0951 g (0,25 mMol) $Pd(OOCCH_3)_2$(2,2'-Bipyridyl), 3 Std. gerührt | 69% d. Th. |

Beispiel 34

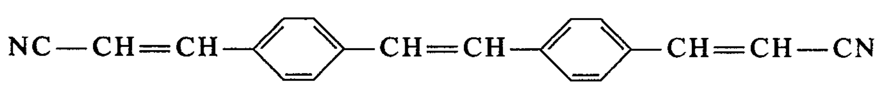

Zu einer Lösung von 9,85 mg (0,044 mMol) Palladiumacetat in 19,7 ml p-Xylol werden unter Argon 0,68 g (4,44 mMol) 4-Vinylzimtsäurenitril, 0,85 g (4,44 mMol) Zimtsäurenitril-4-carbonsäurechlorid und 0,60 g (4,44 mMol) N-Benzyldimethylamin gegeben. Das Gemisch wird während 4 Std. bei 130°C gerührt, auf Raumtemperatur gekühlt und filtriert. Der Niederschlag wird mit Methanol nachgewaschen. Dann wird er in 20 ml heißem N,N-Dimethylformamid gelöst, mit 10 ml Wasser versetzt und auf 0° C gekühlt. Man erhält 0,62 g (50% d. Th.) des obigen optischen Aufhellers; Fp. 220° C.

Das als Ausgangsprodukt verwendete 4-Vinylzimtsäurenitril ist eine neue Verbindung und ebenfalls Gegenstand der Erfindung. Es kann wie folgt hergestellt werden: Zu 6 ml p-Xylol und 6 mg Hydrochinonmonomethyläther werden unter Argon 2,08 g (10 mMol) 4-Bromzimtsäurenitril, 1,85 g (10 mMol) Tri-n-butylamin, 0,0224 g (0,1 mMol) Palladiumacetat und 0,0608 g (0,2 mMol) Tri-o-tolylphosphin gegeben. Das Gemisch wird während 4 Std. bei 10 bar unter Äthylen gerührt. Dann wird das Gemisch bei Raumtemperatur filtriert und mit 40 ml 2N HCl und 40 ml 2N NaOH ausgeschüttelt. Nach dem Trocknen mit Magnesiumsulfat wird das Rohprodukt zweimal aus n-Pentan umkristallisiert. Man erhält 0,22 g (14% d. Th.) 4-Vinylzimtsäurenitril in Form weißer Kristalle; Fp. 45,8° C.

Analyse für $C_{11}H_9N$:
berechnet: C 85,13%, H 5,85%, N 9,02%;
gefunden: C 84,6%, H 5,9%, N 9,0%.

Beispiel 35

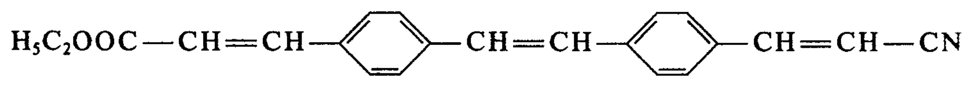

Zu 12,25 mg (0,0549 mMol) Palladiumacetat in 24,5 ml p-Xylol werden unter Argon 0,84 g (5,49 mMol)

4-Vinylzimtsäurenitril, 1,309 g (5,49 mMol) Zimtsäureäthylester-4-carbonsäurechlorid und 0,742 g (5,49 mMol) N-Benzyldimethylamin gegeben. Das Gemisch wird während 5,5 Std. bei 130°C gerührt. Dann wird das Gemisch mit 100 ml Toluol verdünnt und mit 100 ml 2N HCl und 100 ml 2N NaOH ausgeschüttelt. Nach dem Trocknen mit Magnesiumsulfat wird die Lösung eingedampft, und das Rohprodukt wird einmal aus 100 ml Tetrachlorkohlenstoff und einmal aus 50 ml Äthanol umkristallisiert. Man erhält 0,47 g (26% d. Th.) des obigen optischen Aufhellers in Form hellgelber Kristalle; Fp. 155,1°C.

Analyse für $C_{20}H_{14}N_2$:
berechnet: C 80,22%, H 5,82%, N 4,25%;
gefunden: C 80,17%, H 5,79%, N 4,20%.

**Patentansprüche**

1. Verfahren zur Herstellung von Verbindungen der Formel I

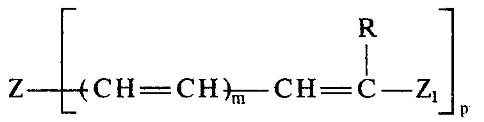

(I)

worin

Z bei p=1 gegebenenfalls substituiertes Phenyl oder Naphthyl und bei p=2 gegebenenfalls substituiertes Phenylen, Naphthylen oder p-Biphenylen,
$Z_1$ gegebenenfalls substituiertes Phenyl oder Naphthyl,
R Wasserstoff oder $C_{1-4}$-Alkyl,
m Null oder die Zahl 1 und
p die Zahl 1 oder 2 bedeuten,

dadurch gekennzeichnet, daß man eine Verbindung der Formel II

$$Z\!-\![(CH\!=\!CH)_m\!-\!CO\!-\!X]_p \qquad \text{(II)}$$

worin X Chlor, Brom oder Jod darstellt und Z, m und p die unter Formel I angegebene Bedeutung haben, in Gegenwart einer Base und unter Zusatz von Palladium-Metall oder von Palladiumverbindungen, die unter den Reaktionsbedingungen phosphorfreie labile Palladium(0)-Verbindungen bilden, als Katalysator mit einer Verbindung der Formel III oder, bei p=2, auch einem Gemisch von zwei verschiedenen Verbindungen der Formel III

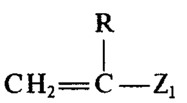

(III)

worin R und $Z_1$ die unter Formel I angegebene Bedeutung haben, umsetzt.

2. Verfahren nach Anspruch 1, dadurch gekennzeichnet, daß man eine Verbindung der Formel II verwendet, worin X Chlor darstellt.

3. Verfahren nach Anspruch 1, dadurch gekennzeichnet, daß man als Säurehalogenid Iso- oder Terephthalsäuredichlorid, 1,4- oder 2,6-Naphthalindicarbonsäuredichlorid, 4,4'-Diphenyldicarbonsäuredichlorid oder eine Verbindung der Formel IIa, IIb, IIc oder IIg

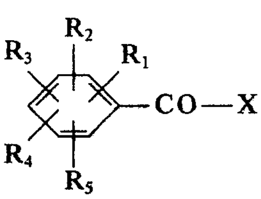

(IIa)

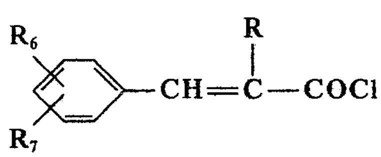

(IIb)

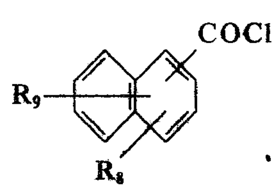

(IIc)

oder

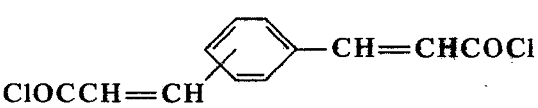

(IIg)

verwendet, worin X Chlor, $R_1$ Wasserstoff, Formyl, Methyl, Methoxy, Cyano, Nitro, Cl, Br, F, J, $-CH=CHCN$, $-CH=CHCOOCH_3$, $-CH=CHCOOC_2H_5$ oder Phenyl, $R_2$ Wasserstoff, Methyl, Methoxy, Cl oder Br, $R_3$ Wasserstoff oder Methoxy, $R_4$ und $R_5$ Wasserstoff, R Methyl und insbesondere Wasserstoff, $R_6$ Wasserstoff, Cl oder Nitro, $R_7$ Wasserstoff, $R_8$ Methyl und insbesondere Wasserstoff und $R_9$ Wasserstoff bedeuten und wobei die $-CH=CHCOCl$-Gruppen in 1,3- oder 1,4-Stellung und die $-COCl$-Gruppe in Formel IIc in 1- oder 2-Stellung gebunden sind.

4. Verfahren nach Anspruch 1, dadurch gekennzeichnet, daß man als Palladiumverbindung

$PdCl_2$, $PdBr_2$, $Pd(OOCCH_3)_2$, $Pd(CH_3COCHCOCH_3)_2$, $Pd(OOCCH_3)_2$ (2,2'-Bipyridyl),

$PdCl_2(NC\text{-}Phenyl)_2$, Bis-(Dibenzylidenaceton)Palladium(0)

oder Bis-(Cyclohexylisonitril)-Palladium(0)

verwendet.

5. Verfahren nach Anspruch 1, dadurch gekennzeichnet, daß man als Palladiumverbindung $PdCl_2$, Palladiumacetat oder Bis-(Dibenzylidenaceton)Palladium(0) verwendet.

6. Verfahren nach Anspruch 1, dadurch gekennzeichnet, daß man die Umsetzung bei einer Temperatur zwischen 0 und 200°C und in Gegenwart eines gegenüber den Reaktionspartnern inerten organischen Lösungsmittels vornimmt.

7. Verfahren nach Anspruch 6, dadurch gekennzeichnet, daß man als Lösungsmittel Anisol, Cylole oder Toluol verwendet.

8. Verfahren nach Anspruch 1, dadurch gekennzeichnet, daß man als Base eine Verbindung der Formel VI

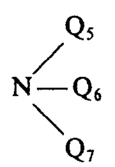

(VI)

verwendet, worin $Q_5$ 4-Chlorbenzyl, 3-Methylbenzyl, 3-Methoxybenzyl oder Benzyl und $Q_6$ und $Q_7$ je Alkyl mit 1–4 C-Atomen bedeuten, oder worin $Q_5$, $Q_6$ und $Q_7$ je Alkyl mit 3–12 C-Atomen darstellen.

9. Verfahren nach Anspruch 1, dadurch gekennzeichnet, daß man als Base N-Benzyldimethylamin, N-Äthylmorpholin oder Tri-n-butylamin verwendet.

10. Verfahren nach Anspruch 1, dadurch gekennzeichnet, daß man eine Verbindung der Formel III verwendet, worin R Methyl und insbesondere Wasserstoff und $Z_1$ Chlorphenyl, Bromphenyl, Zimtsäurenitril, Methylphenyl, Methoxyphenyl, Naphthyl und besonders Phenyl darstellen.

11. Verfahren nach Anspruch 1, dadurch gekennzeichnet, daß man den Katalysator in einer Menge von 0,001 bis 3 Mol-%, bezogen auf die Verbindung der Formel II, verwendet.

12. Verfahren nach Anspruch 1, dadurch gekennzeichnet, daß man als Verbindung der Formel II 4-Brombenzoylchlorid oder Benzoylchlorid und als Verbindung der Formel III 4-Bromstyrol oder Styrol verwendet.

13. 4-Vinylzimtsäurenitril.

**Claims**

1. A process for the preparation of a compound of the formula I

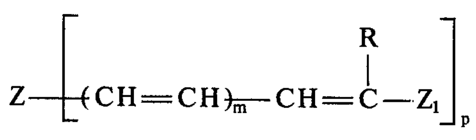

(I)

in which, if p = 1, Z is substituted or unsubstituted phenyl or naphthyl and, if p = 2, Z is substituted or unsubstituted phenylene, naphthylene or p-biphenylene, $Z_1$ is substituted or unsubstituted phenyl or naphthyl, R is hydrogen or $C_{1-4}$-alkyl, m is zero or 1 and p is 1 or 2, wherein a compound of the formula II

$$Z-[(CH=CH)_m-CO-X]_p \quad (II)$$

in which Z, m and p are as defined under formula I and X is chlorine, bromine or iodine, is reacted, in the presence of a base and with the addition, as a catalyst, of palladium metal or of a palladium compound which under the reaction conditions forms a phosphourus-free labile palladium(0)

compound, with a compound of the formula III or, if p = 2, alternatively with a mixture of two different compounds of the formula III

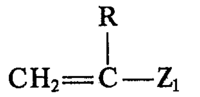

(III)

in which R and $Z_1$ are as defined under formula I.

2. A process according to claim 1, wherein a compound of the formula II, in which X is chlorine, is used.

3. A process according to claim 1, wherein the acid halide used is isophthalic acid dichloride, terephthalic acid dichloride, 1,4- or 2,6-naphthalenedicarboxylic acid dichloride, 4,4'-diphenyldicarboxylic acid dichloride or a compound of one of the formulae IIa, IIb, IIc or IIg

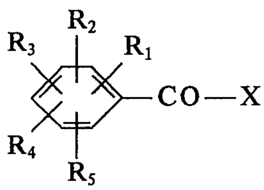

(IIa)

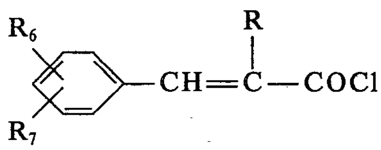

(IIb)

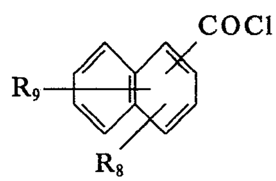

(IIc)

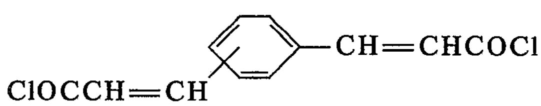

(IIg)

in which X is chlorine, $R_1$ is hydrogen, formyl, methyl, methoxy, cyano, nitro, Cl, Br, F, J, $-CH=CHCN$, $-CH=CHCOOCH_3$, $-CH=CHCOOC_2H_5$ or phenyl, $R_2$ is hydrogen, methyl, methoxy, Cl or Br, $R_3$ is hydrogen or methoxy, $R_4$ and $R_5$ are hydrogen, R is methyl or, especially, hydrogen, $R_6$ is hydrogen, Cl or nitro, $R_7$ is hydrogen, $R_8$ is methyl or, especially, hydrogen and $R_9$ is hydrogen, and the $-CH=CHCOCl$ groups are in the 1,3- or 1,4-position and the $-COCl$ group in formula IIc is in the 1- or 2-position.

4. A process according to claim 1, wherein the palladium compound used is

$PdCl_2$, $PdBr_2$, $Pd(OOCCH_3)_2$, $Pd(CH_3COCHCOCH_3)_2$, $Pd(OOCCH_3)_2$ (2,2'-bipyridyl),

$PdCl_2(NC\text{-phenyl})_2$, bis-(dibenzylidene-acetone)-palladium(0)

or bis-(cyclohexylisonitrile)-palladium(0).

5. A process according to claim 1, wherein the palladium compound used is $PdCl_2$, palladium acetate or bis-(dibenzylidene-acetone)-palladium(0).

6. A process according to claim 1, wherein the reaction is carried out at a temperature of between 0 and 200° C and in the presence of an organic solvent which is inert towards the reactants.

7. A process according to claim 6, wherein the solvent used is anisole, a xylene or toluene.

8. A process according to claim 1, wherein the base used is a compound of the formula VI

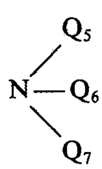

(VI)

in which $Q_5$ is 4-chlorobenzyl, 3-methylbenzyl, 3-methoxybenzyl or benzyl and $Q_6$ and $Q_7$ are each alkyl having 1–4 C atoms, or wherein $Q_5$, $Q_6$ and $Q_7$ are each alkyl having 3–12 C atoms.

9. A process according to claim 1, wherein the base used is N-benzyldimethylamine, N-ethylmorpholine or tri-n-butylamine.

10. A process according to claim 1, wherein a compound of the formula III, in which R is methyl or, especially, hydrogen, and $Z_1$ is chlorophenyl, bromophenyl, cinnamonitrile, methylphenyl, methoxyphenyl, naphthyl or, especially, phenyl, is used.

11. A process accordingto claim 1, wherein the catalyst is used in an amount of 0.001 to 3 mol-%, based on the compound of the formula II.

12. A process according to claim 1, wherein 4-bromobenzoyl chloride or benzoyl chloride is used as the compound of the formula II and 4-bromostyrene or styrene as the compound of the formula III.

13. 4-Vinylcinnamonitrile.

**Revendications**

1. Procédé de préparation de composés de formule (I):

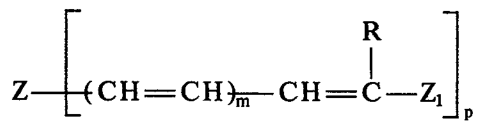

(I)

dans laquelle

- Z si p = 1, est un phényle ou un naphtyle éventuellement substitué, et si p = 2, un groupe phénylène, naphtylène ou p-biphénylène éventuellement substitué,
- $Z_1$ est un phényle ou un naphtyle éventuellement substitué,
- R l'hydrogène ou un alkyle en $C_{1-4}$,
- m le nombre 0 ou 1, et
- p le nombre 1 ou 2,

procédé caractérisé en ce que l'on fait réagir un composé de formule (II):

$$Z-[(CH=CH)_m-CO-X]_p \qquad (II)$$

Z, m et p ayant les mêmes significations que dans la formule (I) et X désignant le chlore, le brome ou l'iode, en présence d'une base et de palladium métal ou de composés du palladium formant dans les conditions réactionnelles des composés de palladium(0) labiles sans phosphore, comme catalyseur, avec un composé de formule (III), ou encore, si p = 2, avec un mélange de deux composés différents de formule (III):

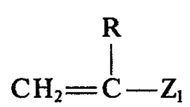

(III)

R et $Z_1$ ayant les mêmes significations que dans la formule (I).

2. Procédé selon la revendication 1 caractérisé en ce que l'on utilise un composé de formule (II) dans lequel X est le chlore.

3. Procédé selon la revendication 1 caractérisé en ce que l'on utilise comme halogénure d'acide le dichlorure de l'acide isophtalique ou téréphtalique, le dichlorure de l'acide 1,4- ou 2,6-naphtalène-dicarboxylique, le dichlorure de l'acide 4,4'-diphényle-dicarboxylique ou bien un composé de formule (IIa), (IIb), (IIc) ou (IIg):

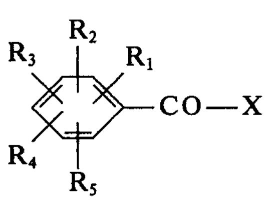

(IIa)

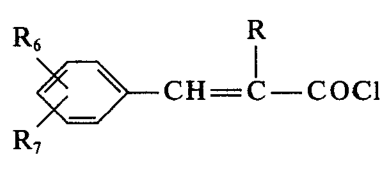

(IIb)

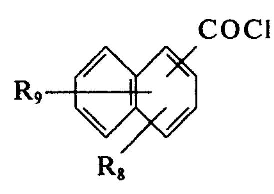

(IIc)

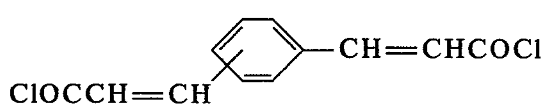

(IIg)

formules dans lesquelles X représente le chlore, $R_1$ l'hydrogène ou un groupe formyle, méthyle, méthoxy, cyano ou nitro, Cl, Br, F ou I, ou bien un groupe $-CH=CHCN$, $-CH=CHCOOCH_3$, $-CH=CHCOOC_2H_5$ ou phényle, $R_2$ représente l'hydrogène, un méthyle ou un méthoxy ou bien Cl ou Br, $R_3$ l'hydrogène ou un méthoxy, $R_4$ et $R_5$ sont chacun l'hydrogène, R est un méthyle et en particulier l'hydrogène, $R_6$ l'hydrogène, le chlore ou un groupe nitro, $R_7$ l'hydrogène, $R_8$ un méthyle et en particulier l'hydrogène et $R_9$ l'hydrogène, et les groupes $-CH=CHCOCl$ occupent les positions 1,3 ou 1,4, et le groupe $-COCl$ de la formule (IIc) occupe la position 1 ou 2.

4. Procédé selon la revendication 1 caractérisé en ce que l'on utilise comme composé du palladium l'un des composés suivants:

$PdCl_2$, $PdBr_2$, $Pd(OOCCH_3)_2$, $Pd(CH_3COCHCOCH_3)_2$, $Pd(OOCCH_3)_2$(2,2'-dipyridyle),

$PdCl_2$(NC-phényle)$_2$, bis-(dibenzylidène-acétone)palladium(0)

ou bis-(cyclohexylisonitrile)palladium(0).

5. Procédé selon la revendication 1 caractérisé en ce que l'on utilise comme composé du palldium $PdCl_2$, l'acétate de palladium ou le bis-(dibenzylidène-acétone)palladium(0).

6. Procédé selon la revendication 1 caractérisé en ce que l'on effectue la réaction à une température de 0 à 200° C en présence d'un solvant organique inerte à l'égard des partenaires réactionnels.

7. Procédé selon la revendication 6 caractérisé en ce que l'on emploie comme solvant l'anisole, un xylène ou le toluène.

8. Procédé selon la revendication 1 caractérisé en ce que l'on utilise comme base un composé de formule (VI):

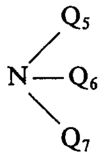

(VI)

dans laquelle $Q_5$ représente un groupe 4-chlorobenzyle, 3-méthylbenzyle, 3-méthoxybenzyle ou benzyle et $Q_6$ et $Q_7$ sont chacun un alkyle en $C_1$ à $C_4$, ou bien dans laquelle $Q_5$, $Q_6$ et $Q_7$ sont chacun un alkyle en $C_3$ à $C_{12}$.

9. Procédé selon la revendication 1 caractérisé en ce que l'on utilise comme base la N-benzyldiméthylamine, la N-éthylmorpholine ou la tri-n-butylamine.

10. Procédé selon la revendication 1 caractérisé en ce que l'on utilise un composé de formule (III) dans lequel R est un méthyle et en particulier l'hydrogène, et $Z_1$ un groupe chlorophényle, bromophényle, cinnamonitrile, méthylphényle, méthoxyphényle, naphtyle ou en particulier phényle.

11. Procédé selon la revendication 1 caractérisé en ce que l'on utilise le catalyseur dans une proportion de 0,001 à 3 moles % par rapport au composé de formule (II).

12. Procédé selon la revendication 1 caractérisé en ce que l'on utilise comme composé de formule (II) le chlorure de 4-bromobenzoyle ou le chlorure de benzoyle, et comme composé de formule (III) le 4-bromostyrène ou le styrène.

13. A titre de produit industriel nouveau, le 4-vinylcinnamonitrile.